# EUROPEAN PATENT APPLICATION

(11) **EP 3 000 892 A1**
(43) Date of publication of application: **30.03.2016**
(21) Application number: 14461571.3
(22) Date of filing: 26.09.2014
(51) Int. Cl.: C12P 5/02

(54) **Use of a microbiological consortium and a microbiological medium for the production of biomethane**

(71) Applicant: Politechnika Lódzka, 90-924 Lódz (PL)
(72) Inventor: Zieminski, Krzysztof, 93-355 Lódz (PL)
(74) Representative: Rumpel, Alicja

(57) **Abstract**

Application of microbiological consortium and microbiological culture medium for the production of biomethane, especially as engine fuel. The microbiological consortium was isolated from a natural environment, rich in sulphur, and immobilised in a fermentation tank under anaerobic conditions. A microbiological culture medium contains in its composition KH₂PO₄ (from 0.1 to 50 g/L), MgCl×6H₂O (from 0.01 to 50 g/L), Ca(NO₃)×4H₂O (from 0.1 to 1000 g/L) and FeSO₄×7H₂O (from 0.01 to 50 g/L) and tap water.

## Description

The invention concerns the application of microbiological consortium and microbiological culture medium for the production of biomethane, especially as engine fuel

The development of road transport causes a continuous growth of emissions of by-products from the combustion process of petrol or diesel fuel. The emissions go into the atmosphere, providing a source of contamination for the natural environment. Therefore, the, so called, clean engine fuels, such a biomethane, while being a more environment-friendly alternative to mining fuels, become more and more important for the resulting reduction of contaminations, released to the natural environment. A significant advantage of clean engine fuels is their independence vs. the depleting natural resources from the unsustainable sources.

The term "biomethane" means a purified and enriched biogas, upgraded to the parameters of natural gas. Biogas is generated in result of the activity of anaerobic bacteria, causing degradation of organic substances. Biogas mixture usually contains approximately 55-85% of methane (CH₄), 30-45% of carbon dioxide C0₂), 0.01-5% of hydrogen sulphide (H₂S) plus low concentrations of nitrogen, oxygen and steam. The main cause of reduced fuel calorific value is the presence of contaminations, especially of CO₂ and H₂S.

In biomethane production, technologies are preferred with no burdens for the natural environment. They provide an attractive option for the low capital outlays and the lack of negative effects on the natural environment. The main assumptions of the biotechnological methods include having appropriate, specially selected microorganisms plus the knowledge of conditions in which they may effectively be used. Actually, the key problem is the lack of highly effective methods of biological biogas purification on industrial scale. Another problem, associated with the application of biological methods is the presence of elementary sulphur in the installation on which desulphurisation is performed. Sulphur compounds are oxidised to elementary sulphur which is then deposited in the system and has to be regularly removed.

Patent description No. WO2012061933 presents a system of H₂S eliminating bioreactor, where a psychotrophic anaerobic bioreactor was applied, operating in microaerophilic conditions, where the microconsortium is built by bacteria of an active anaerobic sediment, while the organic substrate is made of animal, agricultural, communal, agricultural-food-processing or industrial wastes or of their mixture. In this purification process, a special reaction provides an elementary sulphur product, which is then converted into deposits. Since it is organic wastes, which provide the source of carbon for the microorganisms, in result of natural selection heterotrophic microorganisms dominate, which do not demonstrate any ability of binding with CO₂, thus being unable to remove it from biogas. What is more, oxygen injection dissolves biogas. Patent description No. EP1604727 depicts a biogas desulphurisation method in presence of sulphate and iron ions by means of halotolerant microorganisms, with the use of nutrient components in the culture medium, such as (NH₄)₂SO₄, KCI, K₂HPO₄, MgSO₄, Ca(NO₃)₂. In this purification process, a special reaction provides a product in the form of elementary sulphur. Patent description No. WO2008131034 presents a bioscrubber system to remove H₂S with hydrogen sulphide oxidising bacteria, acquired from hot springs and the application of liquid wastes as the culture medium for microorganisms. In such a system, in result of natural selection, heterotrophic microorganisms dominate, which do not demonstrate any ability of binding with CO₂, thus being unable to remove it from biogas. H₂S is oxidised into sulphur and SO₄²⁻. In result of the process, pH level decreases, what has then to be compensated.

The application of the microbiological consortium and the microbiological culture medium for biomethane production, in particular as engine fuel, according to this invention, involves the use of microorganisms, able to decompose hydrogen sulphide in a fermentation tank with subsequent assimilation of carbon from CO₂ for the application of microbiological culture medium without organic carbon source, containing in its composition KH₂PO₄ (from 0.1 to 400 g/L), MgCl×6H₂O (from 0.01 to 200 g/L), Ca(NO₃)×4H₂O (from 0.1 to 2000 g/L) and FeSO₄×7H₂O (from 0.01 to 50 g/L) and water, as well as preferably a mixture of trace elements (per 1L of solution): ZnSO4 - from 0.1-200.0 g, , MnCl2 - from 0.1 - 100.05 g, CoCl2 - from 0.1 - 50.0 g, CuSO4-from 0.1 - 50.0g, (NH4)2MoO4 - from 0.1 - 50.0 g

The culture medium, according to this invention, is used for sprinkling biological sediments, on which microorganisms are immobilised in the fermentation tank under anaerobic conditions. The volumetric flow intensity of the biological sediment sprinkling medium preferably ranges within 5 L/h to 500 L/h per one cubic meter of the biological system. It is preferable that pH of the culture medium is 5-8, preferably not below 5.5 and not above 7.0-7.5. The microbiological consortium, according to this invention, was acquired from a natural environment, rich in sulphur. The microbiological consortium includes bacteria of α-Proteobacteria, β-Proteobacteria and γ-Proteobacteria, belonging to the following orders: Burkholderiales, Xanthomonodales, Rhodocyclales, Hydrogenophilales, Rhizobiales, Sphingomonodales, Enterobacteriales, Methylophilales , Caulobacteriales , Neisseriales , Rhodospirrales , Pseudomonadales , Oceanospirillales , Methylococcales, belonging to the following genera: Rhodanobacter, Thiomonas, Ralstonia, Thermomonas, Simplicispira, Thiobacillus, Propionivibrio, Sphingomonas, Oxalobacter, Denitratisoma, Thermomonas, Parvibaculum, Simplicispira, Hylemonella, Comamonas, Citrobacter, Pedomicrobium, Methylotenera, Alcanivorax, Acinetobacter, Aminobacter, Methylomicrobium, Methylomonas, Acidovorax, Azospira, Pseudomonas, Candidatus Blochmannia and Sphingopyxis.

Preferably, the microbiological consortium contains bacteria of the following species: Citrobacter werkmanii, Denitratisoma oestradiolicum, Denitrobacter permanent, Limnohabitans parvus, Luteibacter anthropi, Methylotenera versatilis, Oxalobacter vibrioformis, Propionivibrio dicarboxylicus , Ralstonia insidiosa, Rhodanobacter fulvus, Rhodanobacter ginsenosidimutans, Rhodanobacter lindaniclasticus, Thermomonas dokdonensis, Thermomonas Fusa , Thiobacillus thioparus, Thiobacillus thiophilus, Thiomonas intermedia, Thiomonas perometabolis, Vogesella perlucida, Ralstonia detusculanense, Aminobacter aminovorans and Comamonas composti.

Moreover, the biological consortium may also include bacteria, belonging to the following classes: Actinobacteria, Sphingobacteria, Verrucomicrobiae, Holophagae, Planctomycetia, Verrucomicrobiae, Clostridia, ε-Proteobacteria, Δ-Proteobacteria, Flavobacteria, Cyanobacteria, Bacteriodetes which belong to the following orders: Actinomycetales , Sphingobacteriales , Holophagales, Bacteroidales, Verrucomicrobiales, Gemmatales , Campylobacteriales, Desulfurellales, Flavobacteriales, Stigonematales, belonging to the following genera: Geothrix, Rhodocuccus, Prosthecobacter, Singulisphaera, Cellulomonas, Arthrobacter, Desulfurella, Segetibacter, Chryseobacterium, belonging to the following species: Rhodothermus clarus, Arthrobacter halodurans, Arthrobacter psychrolactophilus, Arthrobacter stackebrandtii, Chryseobacterium soli and Segetibacter aerophilus.

The culture medium components are added to water of predefined volume and the resulting solution is mechanically stirred for approximately 15 minutes.

Biogas, produced in the process of methane fermentation, is pumped into a pipeline and then to the biological desulphurisation system, based on a sprinkled biofilter, used an element of the industrial installation. The flowing gas volume is controlled with a flow control (13). A biological system, consisting of a porous filtration material, enabling formation of specific biofilm with activity level, ensuring biogas purification, is placed in the biofilter column. The biofilter system is sprinkled from top with the culture medium. The culture medium, dripping through the biofilter down to the column bottom, is drained into the tank. The culture medium is then returned to the system with a pump, flowing through a flow meter up to the sprinklers.

The advantage of this invention is 97-100% H₂S reduction in industrial conditions, allowing to achieve biogas parameters comparable with the natural gas parameters. What is more, as there is no carbon in the culture medium, the microorganisms collect is from CO₂, what contributes to removal of this ballast material from biogas by approximately 40%. Another advantage of this invention is a minimised sulphur production in the biogas purification process and a limited deposit volumes in the installation, on which the process is carried out. The process does not reduce pH, either, what eliminates the need for later pH correction.

The subject of the invention has been explained in a more detail in the application example.

### Example 1:

### Example: preparation of microbiological culture medium

A 600 L container was filled with tap water, poured in a continuous stream. While pouring the tap water, the following solutions were subsequently added: 5 g of FeSO₄×7H₂O, solved in 1 L of tap water, 100 g of MgCL₂×6H₂O, solved in 1 L of tap water and 250 g of KH₂PO₄, solved in 2 L of tap water. When approximately 400 L of water was poured, a solution of 250 g of Ca(NO₃₎₂×6H₂O was added and water filling was continued up to the nominal volume. The whole volume was mechanically stirred for approximately 15 minutes.

### The structure and functions of biomethane production installation

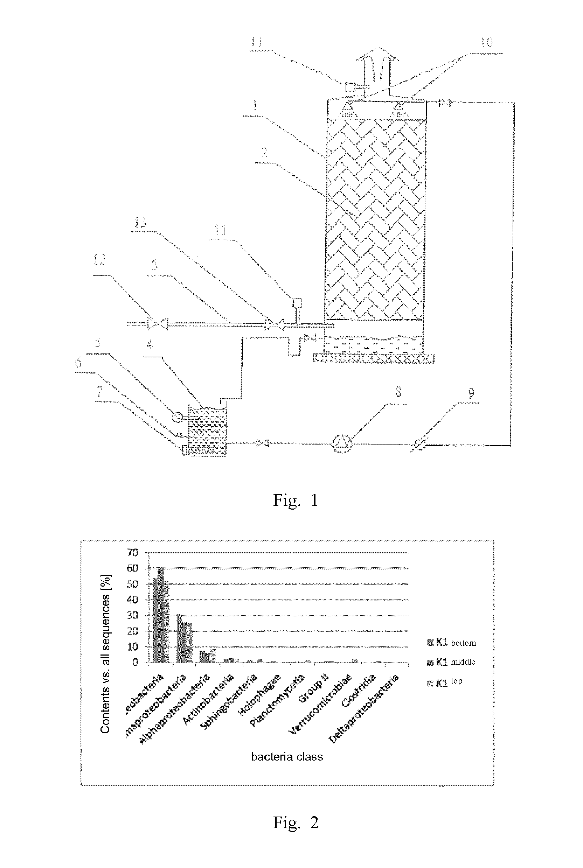

This invention employed a system, based on a sprinkled biofilter, working as an element of the industrial installation. Contaminated gas was delivered from the bottom of the system, while a recirculating culture medium sprinkled the column packing from the top in a volume of 30 L/h. The microorganisms, making the consortium of microorganisms, isolated from natural environments, rich in sulphur, able for effective biogas purification, were immobilised on the carrier. The process was carried out in anaerobic conditions. The installation for biogas purification has been illustrated in the drawing, where fig. 1 presents its structure. Biogas, produced in the process of methane fermentation, was pumped under 300 ppm pressure into the pipeline (3) and then to the biological desulphurisation system. The flowing gas volume was controlled with a flow control (13). H₂S measurement and recording, both at inlet and outlet from the biofilter, was performed by H₂S concentration sensors (11) A biological system, consisting of a porous filtration material (2), enabling formation of specific biofilm with activity level, ensuring biogas purification, was placed in the biofilter column (1). The biofilter system was sprinkled from top (10) with the culture medium in a volume of 30 L/h. The culture medium, dripping through the biofilter down to the column bottom, was drained into the tank (4). The culture medium was then returned to the system with a pump (8), flowing through a flow meter (9) up to the sprinklers (10). The culture medium tank (4) had a heater (7) with a temperature sensor (5) to maintain constant temperature in the tank, as well as a culture medium level sensor (6). Biogas flow to the installation was cut off by the valve (12). Desulphurisation efficiency was 98-99%.

### Microbiological consortium analysis

The microbiological consortium at three column levels (bottom, middle level, top) was characterised after installation and after 45 days of operation by sequencing the products of PCR amplification.

The microbiological consortium at three column levels (bottom, middle level, top) was characterised after installation and after 45 days of operation by sequencing the products of PCR amplification.

DNA from samples was isolated by means of a Bead-Beat Micro Gravity DNA isolation kit of A&A Biotechnology (ref. No. 106-20). DNA quantification was carried out with a Quant-iT PicoGreen dsDNA Assay kit of Molecular Probes (ref. No. P11496) by fluorescence measurement. DNA amplification reactions for sequencing purposes were carried out acc. to the description, presented in the publication of Caporaso et al. (2012) The sequencing was done with a MiSeq v2 2x250nt Reagent Kit of iLLUMINA (ref. No. 15033625), using the starters, presented in the report of Caporaso et al. (2012) The sequencing results were automatically analysed on a MiSeq device of Illumina, using the MiSeq Reporter (MSR) software v2.4, 16S Metagenomics protocol. The analysis consisted of three stages: i) automatic demultiplexing of samples, ii) generating of fastq files with raw recordings, iii) classification of paired-end readouts in particular taxonomic categories. The 16S Metagenomics protocol ensured a classification of readouts to the species level, on the basis of Greengenes v13_5 reference sequence data, modified by the Illumina Company. The preparation of the reference database of reference sequences included: i) defiltration of the sequences below 250 base pairs; ii) defiltration of the sequences with more than 50 degenerated bases (M, R, W, S, Y, K, V, H, D, B, N); iii) defiltration of incompletely classified sequences (no classification to the level of genus or species).

Fig. 2. A list of percentage shares of particular bacteria classes at the three levels of the column packing - the bottom (blue colour), the middle level (red colour) and the top (green colour) at column operation starting point.

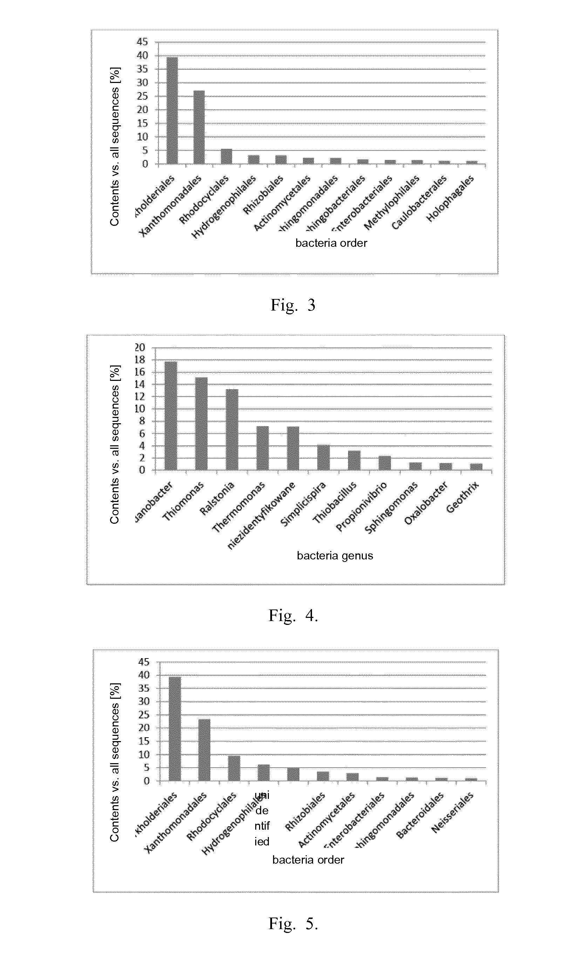

Fig. 3. Percentage shares of particular bacteria orders in the bottom part of the column packing at column operation starting point.

Fig. 4. Percentage shares of particular bacteria genera in the bottom part of the column packing at column operation starting point.

Fig. 5. Percentage shares of particular bacteria orders in the middle part of the column packing at column operation starting point.

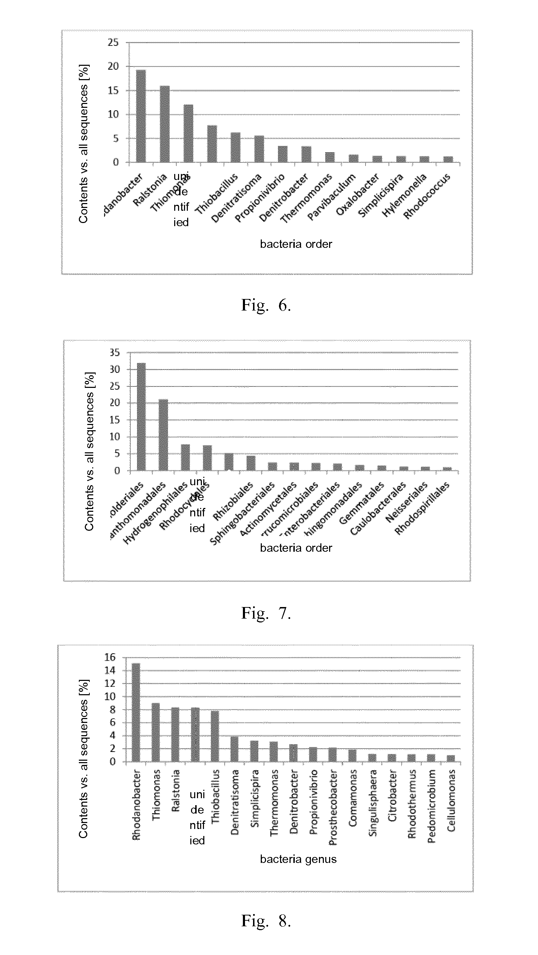

Fig. 6. Percentage shares of particular bacteria genera in the middle part of the column packing at at column operation starting point.

Fig. 7. Percentage shares of particular bacteria orders in the upper part of the column packing at column operation starting point.

Fig. 8. Percentage shares of particular bacteria genera in the upper part of the column packing at column operation starting point.

**Table 1. List of species in the highest number in particular parts of the column packing at column operation starting point.**

| bacteria species | Percentage shares in particular column parts | | |
|---|---|---|---|
| | the bottom | the middle level | the top |
| unidentified | 35.68 | 33.81 | 37.81 |
| Citrobacter werkmanii | 0.6 | 0.15 | 0.76 |
| Denitratisoma oestradiolicum | 1.98 | 5.54 | 3.84 |
| Denitrobacter permanens | 0.19 | 0.94 | 0.64 |
| Limnohabitans parvus | 0.86 | 0.24 | 0.8 |
| Luteibacter anthropi | 0.59 | 0.69 | 0.55 |
| Methylotenera versatilis | 0.65 | 0.36 | 0.31 |
| Oxalobacter vibrioformis | 1.14 | 1.33 | 0.9 |
| Propionivibrio dicarboxylicus | 3.25 | 3.41 | 2.23 |
| Ralstonia insidiosa | 12.6 | 15.07 | 7.82 |
| Rhodanobacter fulvus | 1.02 | 0.47 | 0.97 |
| Rhodanobacter ginsenosidimutans | 2.33 | 1.4 | 2.16 |
| Rhodanobacter lindaniclasticus | 4.08 | 5.15 | 3.38 |
| Rhodothermus clarus | 0.26 | 0.04 | 1.13 |
| Thermomonas dokdonensis | 2.41 | 0.69 | 0.86 |
| Thermomonas fusca | 1.53 | 0.61 | 0.97 |
| Thiobacillus thioparus | 0.14 | 0.08 | 1.17 |
| Thiobacillus thiophilus | 2.58 | 5.44 | 4.97 |
| Thiomonas intermedia | 14.14 | 10.91 | 8.41 |
| Thiomonas perometabolis | 0.65 | 0.96 | 0.44 |
| Vogesella perlucida | 0.56 | 0.69 | 0.84 |

The microbiological consortium was also characterised after 45 days of the column operation.

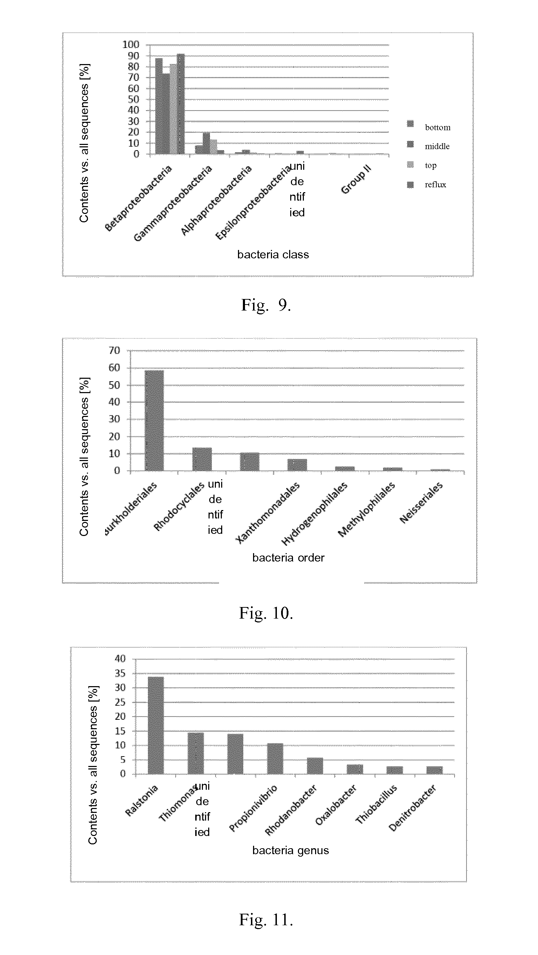

Fig. 9. A list of percentage shares of particular bacteria classes at the three levels of the column packing - the bottom (blue colour), the middle level (the red colour) and the top (green colour) and in the reflux (violet colour) after 45 days of column operation.

Fig. 10. Percentage shares of particular bacteria orders in the bottom part of the column packing after 45 days of column operation.

Fig. 11. Percentage shares of particular bacteria genera in the bottom part of the column packing after 45 days of column operation.

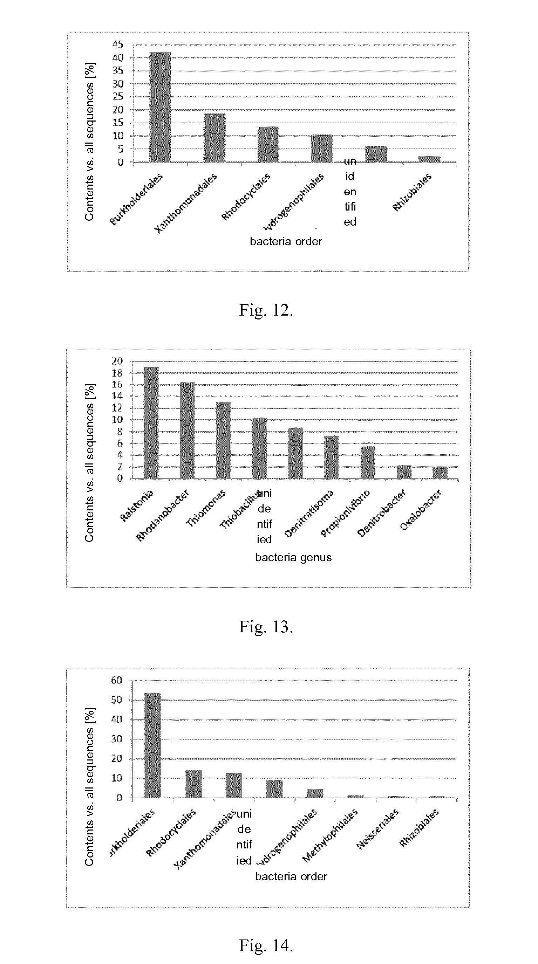

Fig. 12. Percentage shares of particular bacteria orders in the middle part of the column packing after 45 days of column operation.

Fig. 13. Percentage shares of particular bacteria genera in the middle part of the column packing after 45 days of column operation.

Fig. 14. Percentage shares of particular bacteria orders in the upper part of the column packing after 45 days of column operation.

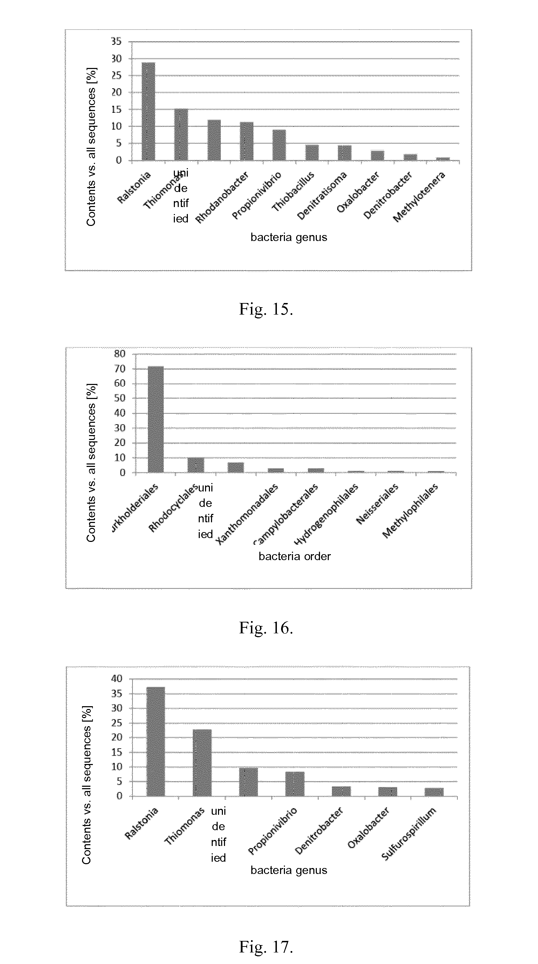

Fig. 15. Percentage shares of particular bacteria genera in the upper part of the column packing after 45 days of column operation.

Fig. 16. Percentage shares of particular bacteria orders in the column reflux after 45 days of column operation.

Fig. 17. Percentage shares of particular bacteria genera in the column reflux after 45 days of column operation.

**Table 2. List of species in the highest number in particular parts of the column packing after 45 days of column operation.**

| bacteria species | Percentage shares in particular column parts | | | |
|---|---|---|---|---|
| | the bottom | the middle level | the top | reflux |
| unidentified | 23.32 | 26.36 | 23.99 | 20.19 |
| Denitratisoma oestradiolicum | 1.59 | 1.92 | 4.27 | 1 |
| Denitrobacter permanens | 1.02 | 0.79 | 0.6 | 1.07 |
| Methylotenera versatilis | 1.24 | 0.58 | 0.84 | 0.58 |
| Oxalobacter vibrioformis | 3.28 | 1.92 | 2.81 | 3.12 |
| Propionivibrio dicarboxylicus | 10.74 | 5.48 | 8.94 | 8.32 |
| Ralstonia detusculanense | 1.57 | 0.9 | 1.18 | 1.14 |
| Ralstonia insidiosa | 31.84 | 17.85 | 27.26 | 35.69 |
| Rhodanobacter lindaniclasticus | 2.34 | 6.47 | 4.93 | 0.81 |
| Thiobacillus thiophilus | 2.38 | 9.39 | 4.18 | 0.89 |
| Thiomonas intermedia | 13.4 | 11.9 | 14.23 | 21.08 |
| Thiomonas perometabolis | 0.92 | 0.94 | 0.8 | 1.37 |

## Claims

1. An application of microbiological consortium and of microbiological culture medium for biomethane production, in particular, as engine fuel, is characteristic in that the microbiological consortium was isolated from natural environments, rich in sulphur, and immobilised in a fermentation tank under anaerobic conditions, while the microbiological culture medium contains in its composition KH₂PO₄ (from 0.1 to 50 g/L), MgCl×6H₂O (from 0.01 to 50 g/L), Ca(NO₃)×4H₂O (from 0.1 to 1000 g/L) and FeSO₄×7H₂O (from 0.01 to 50 g/L) plus water.

2. The application of microbiological consortium and of microbiological culture medium for the production of biomethane, especially as engine fuel, according to claim 1, characteristic in that it contains a mixture of trace elements (per 1 L of solution): ZnSO4 - from 0.1 - 200.0 g, , MnCl2 - from 0.1 - 100.05 g, CoCl2 - from 0.1 - 50.0 g, CuSO4 - from 0.1-50.0g, (NH4)2MoO4 - from 0.1 - 50.0 g

3. The application of microbiological consortium and of microbiological culture medium for the production of biomethane, especially as engine fuel, according to claim 1 or 2, characteristic in that the preferable volumetric flow intensity of the biological system sprinkling medium preferably ranges within 5 L/h to 500 L/h per one cubic meter of the biological system.

4. The application of microbiological consortium and of microbiological culture medium for the production of biomethane, especially as engine fuel, according to claim 1 or 2 or 3 characteristic in that pH of the culture medium is 5-8, preferably not below 5.5 and not above 7.0-7.5.

5. The application of microbiological consortium and of microbiological culture medium for the production of biomethane, especially as engine fuel, according to claim 1 or 2 or 3 or 4, characteristic in that the microbiological consortium includes bacteria of α-Proteobacteriae and/or β-Proteobacteriae and/or γ-Proteobacteriae, belonging to the following orders:Burkholderiales and/or Xanthomonodales and/or Rhodocyclales and/or Hydrogenophilales and/or Rhizobiales and/or Sphingomonodales and/or Enterobacteriales and/or Methylophilales and/or Caulobacteriales and/or Neisseriales and/or Rhodospirrales and/or Pseudomonadales and/or Oceanospirillales and/or Methylococcales, belonging to the following genera: Rhodanobacter and/or Thiomonas and/or Ralstonia and/or Thermomonas and/or Simplicispira and/or Thiobacillus and/or Propionivibrio and/or Sphingomonas and/or Oxalobacter and/or Denitratisoma and/or Thermomonas and/or Parvibaculum and/or Simplicispira and/or Hylemonella and/or Comamonas and/or Citrobacter and/or Pedomicrobium and/or Methylotenera and/or Alcanivorax and/or Acinetobacter and/or Aminobacter

6. The application of microbiological consortium and of microbiological culture medium for the production of biomethane, especially as engine fuel, according to claim 11 or 2 or 3 or 4 or 5, characteristic in that biogas, produced in the process of methane fermentation, is pumped into a pipeline and then to the biological desulphurisation system, based on a sprinkled biofilter, used an element of the industrial installation, while the flowing gas volume is controlled with a flow control, where a biological system, consisting of a porous filtration material, enabling formation of specific biofilm with activity level, ensuring biogas purification, is placed in the biofilter column, the biofilter system is sprinkled from top with the culture medium and the culture medium, dripping through the biofilter down to the column bottom, is drained into the tank and returned with a pump, flowing through a culture medium flow meter up to the sprinklers.
